# EUROPEAN PATENT SPECIFICATION

(11) **EP 1 779 827 B1**
(45) Date of publication and mention of the grant of the patent: **03.03.2010**
(21) Application number: 05780219.1
(22) Date of filing: 12.08.2005
(51) Int. Cl.: A61F 13/15

(54) **DISPOSABLE PANTS**
EINWEG-SLIPS
CULOTTES JETABLES

(30) Priority: 17.08.2004 JP 2004237235
(43) Date of publication of application: 02.05.2007
(73) Proprietor: Livedo Corporation, Shikokuchuo-shi, Ehime 799-0122 (JP)
(72) Inventor: FUJIOKA, M., Tokushima Sadamitsu Plant Livedo Corp, Mima-gun, Tokushima 779-4104 (JP)
(74) Representative: Müller-Boré & Partner Patentanwälte
(86) International application number: PCT/JP2005/014828
(87) International publication number: WO 2006/019051

(56) References cited:
- EP-A- 1 110 529
- EP-A1- 0 648 483
- FR-A- 2 764 185
- FR-A1- 2 485 893
- GB-A- 2 267 024
- JP-A- 5 317 356
- JP-A- 8 229 072
- JP-A- 2003 528 649
- JP-A- 2003 528 650
- US-A1- 2002 040 214
- US-A1- 2003 055 389

## Description

### Technical Field

The present invention relates to disposable pants usable as both pants and a diaper.

### Background Art

As conventional disposable pants of this type, there is a technique described in Japanese Patent Application Laid-Open No. 5-317356. The pants described in this gazette are provided with breaking lines on both left and right sides of a front section to allow the front section to be separated at the breaking lines as well as adhesive pieces bonded to bonding parts on the left and right sides of the front section and a rear section. When the front section is separated at the breaking lines, the front section and rear section are fastened by the left and right adhesive pieces.

However, with the aforementioned conventional technique, adhesive parts provided on the adhesive pieces may adhere to those places other than a specified position of the pants or places such as garments other than the pants, making them difficult to handle.

Further, US 2003/0055389 A1 discloses an absorbent garment including a first front body panel and a second rear body panel. One or more laterally extending elastic elements are secured to each of the first and second body panels. The front body panel has a deactivated area wherein the elastic elements are severed, chopped or otherwise deactivated. The deactivated area is formed along a center portion of the front body panel. One or more leg elastic elements can be secured along inner terminal edges of the body panels and an absorbent composite to form a gasket with the leg of the user at the leg opening formed by the absorbent garment.

US 2002/0040214 A1 shows an absorbent product such as a nappy, an incontinence pad, or the like. The product has longitudinal side edges, transverse end edges, a first end section and a second end section and a crotch section lying between the end sections. The product has a fastening system including at least one fastening tab arranged at a side edge of the product, on the first end section, and including a first fastening member and a second fastening member arranged on the second end section of the product and arranged so as to interact with the first fastening member in order to bind together the product into a briefs-like shape. The fastening tab constitutes a part of a material strip which extends in the transverse direction of the product and is fixed permanently to the product, at least at its ends. The fastening tab is demarcated from the rest of the material strip by a tear line extending substantially transversely to the direction of extent of the material strip.

EP 1 110 529 A1 discloses a nappy having an impermeable outer layer, a permeable inner layer and an absorbent pad between them, has a waistband attached to the rear portion of the outer layer and equipped with end fastenings. Prior to use the end fastenings are folded along transverse lines so they do not project beyond the edges of the nappy. The fastenings can be held in the folded position by a temporary adhesive or thermal or ultrasound welding points. The waistband can be made, for example, from non-woven, woven or knitted fabric, optionally combined with a plastic film layer.

Further, GB 2 267 024 A shows a diaper wherein respective laterally opposite side portions of front and rear bodies and base ends of respective fastening flaps are all bonded together along bond lines, and adjacent these bond lines, the front body is provided with cutting lines along which laterally opposite side portions of the front body may be torn off from the corresponding opposite side portions of the rear body. The diaper is thus usable selectively either as pants type diaper with front and rear bodies bonded together along their laterally opposite side portions, or as the open type diaper with the front and rear bodies separated at their side portions but connectible with each other by the fastening flaps when the diaper is used.

### Disclosure of Invention

To solve the aforementioned problem, the present invention has an object to provide disposable pants capable of preventing adhesive pieces from undesirably adhering to those places other than a specified position when the adhesive pieces are not used.

In a first aspect of the present invention, disposable pants comprising a front abdominal section and a rear section joined almost annularly and a crotch section provided to be joined between the front abdominal section and rear section, the crotch section being provided with an absorber, left and right breaking parts for breaking the front abdominal section being provided on both left and right sides of an area of the front abdominal section to which the crotch section is joined, comprise: a first adhesive part provided in at least part of a central area positioned between the left and right breaking parts on an exterior side of the front abdominal section; left and right adhesive pieces bonded to laterally outward sides of the left and right breaking parts in the front abdominal section; and second adhesive parts provided on a skin-facing side of the left and right adhesive pieces to be attached/detached to/from the first adhesive part and having functions of both pants and a diaper at once, wherein, when a function of pants is used, a portion of each of the left and right adhesive pieces on which said second adhesive parts are provided is folded on the skin-facing side so as to prevent the adhesion of said second adhesive parts, and wherein in such a folded state, temporarily fixing parts provided on the respective skin-facing side of said portions holds the corresponding adhesive pieces in said folded state.

Further, according to a second aspect of the present invention, in the aforementioned first aspect, the temporarily fixing parts bond portions of the left and right adhesive pieces folded on the skin-facing side with such a strength that they can be peeled.

Further, according to a third aspect of the present invention, in any one of the aforementioned aspects, elastic stretchable means is provided in an area other than the central area of the front abdominal section and an area around the hips of the rear section.

According to the aforementioned first aspect, when the left and right adhesive pieces are not used, portions of the adhesive pieces where the second adhesive parts are provided can be folded at pleats on the skin-facing side. Therefore, when the left and right adhesive pieces are not used, the adhesive pieces can be prevented from undesirably adhering to those places other than a specified position of the disposable pants or adhering to garments and the like, which provides easy handling.

Further, according to the invention described in the aforementioned first aspect, when the adhesive pieces are not used, the temporarily fixing parts reliably allow the left and right adhesive pieces to be held in the state folded at the pleats on the skin-facing side.

Further, according to the invention described in the aforementioned second aspect, when the adhesive pieces are used, the temporarily fixing parts can easily be peeled to expand the adhesive pieces.

Further, according to the invention described in the aforementioned third aspect, since the waist area of the disposable pants can easily be stretched, the disposable pants can easily be raised/lowered with the left and right adhesive pieces adhering to the first adhesive part.

These and other objects, features, aspects and advantages of the present invention will become more apparent from the following detailed description of the present invention and accompanying drawings.

### Brief Description of Drawings

[Fig. 1] is a front view of disposable pants according to an embodiment of the present invention.
[Fig. 2] is a diagram in which a right adhesive piece of the disposable pants shown in Fig. 1 is broken.
[Fig. 3] is a diagram showing the state in which a right breaking part of the disposable pants shown in Fig. 1 is broken to expand a right front abdominal part.
[Fig. 4] is a diagram showing the state in which a left breaking part of the disposable pants shown in Fig. 3 is broken to expand a left front abdominal part.
[Fig. 5] is a diagram showing the state in which a crotch section of the disposable pants shown in Fig. 4 is expanded.
[Fig. 6] is a diagram of the disposable pants shown in Fig. 5 as viewed from the opposite side to Fig. 5.
[Fig. 7] is a sectional view taken along a line C1-C1 of the disposable pants shown in Fig. 1.
[Fig. 8] is an enlarged view of part of Fig. 7.
[Fig. 9] is a sectional view taken along a line C2-C2 of the disposable pants shown in Fig. 5.
[Fig. 10] is a diagram showing the state in which the left and right adhesive pieces adhere to a central front abdominal part.
[Fig. 11] is a diagram showing a variant of the left and right adhesive pieces. Best Mode for Carrying Out the Invention

### <General Description>

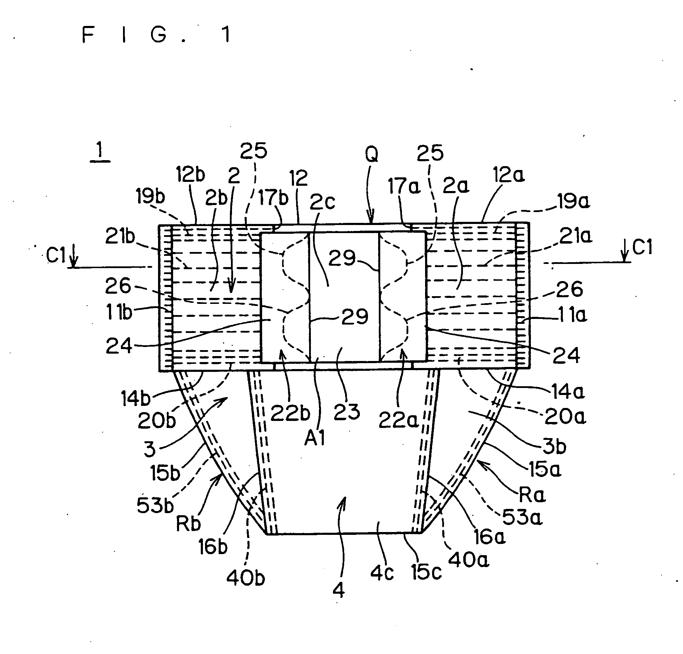

With reference to Figs. 1 to 10, disposable pants 1 according to an embodiment of the present invention will be described. The disposable pants 1, as shown in Figs. 1 to 10, are configured to comprise a front abdominal section 2 and a rear section 3 joined almost annularly and a crotch section 4 provided to be joined between the front abdominal section 2 and rear section 3, and are usable as both pants and a diaper. In the description of the disposable pants 1, the left and right shall indicate the left hand side and right hand side as viewed from a wearer.

The front abdominal section 2 and rear section 3 refer to portions of the disposable pants 1 that mainly face a front abdominal area and an area on the back of a wearer. Left and right edges of the front abdominal section 2 and left and right edges of the rear section 3 are bonded to each other, and the front abdominal section 2 and rear section 3 are thereby joined almost annularly. Accordingly, a left side bonding part 11a and a right side bonding part 11b for bonding the left and right edges of the front abdominal section 2 and left and right edges of the rear section 3 are formed on left and right edges of the disposable pants 1. Bonding at these side bonding parts 11a and 11b is created either by bonding with an adhesive such as a hot melt adhesive or ultrasonic welding (or heating welding), or by both of them in combination.

The crotch section 4 indicates a portion of the disposable pants 1 that mainly faces the crotch of a wearer, having a front crotch part 4a and a rear crotch part 4b joined to the front abdominal section 2 and rear section 3, respectively. In this embodiment, the front crotch part 4a and rear crotch part 4b of the crotch section 4 are joined to the front abdominal section 2 and rear section 3 by an adhesive such as a hot melt adhesive. As a variant, the crotch section 4 may be formed integrally by a member connected to one or both of the front abdominal section 2 and rear section 3.

A waist opening Q is formed by upper edges 12 and 13 of such front abdominal section 2 and rear section 3 joined almost annularly. A left leg opening Ra is formed by a lower edge 14a of a left front abdominal part 2a of the front abdominal section 2, a sloped edge 15a on the left lower side of the rear section 3 and a left edge 16a of the crotch section 4. A right leg hole Rb is formed by a lower edge 14b of a right front abdominal part 2b of the front abdominal section 2, a sloped edge 15b on the right lower side of the rear section 3 and a right edge 16b of the crotch section 4.

### <Front Abdominal Section>

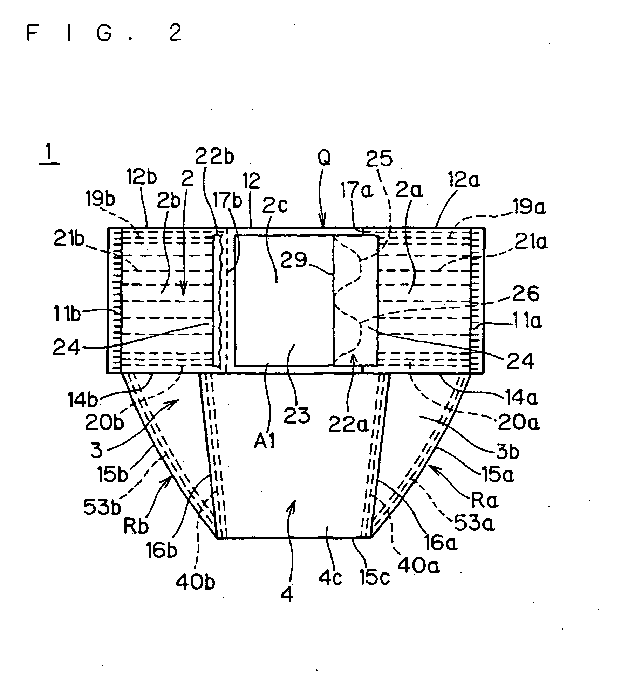

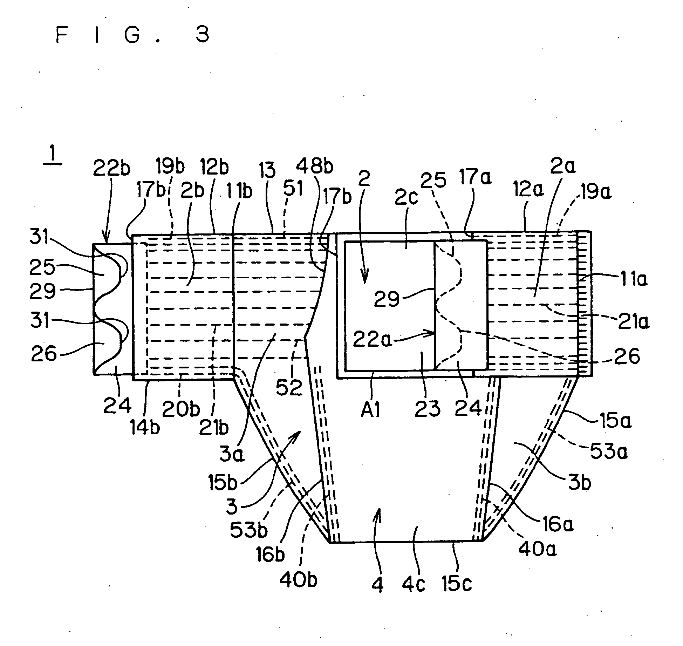

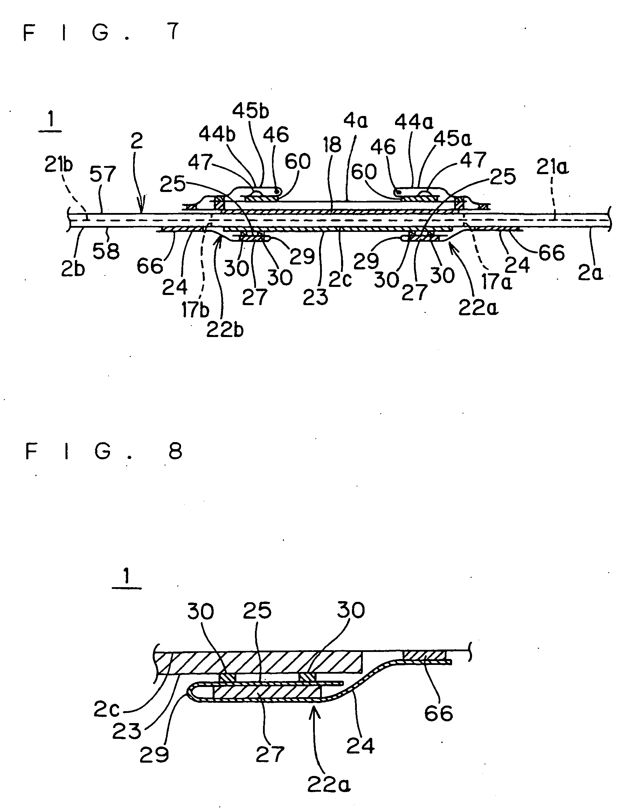

The front abdominal section 2 is, as shown in Fig. 1, of almost laterally long rectangular shape in plan view as an overall configuration, and includes the left front abdominal part 2a, right front abdominal part 2b and a central front abdominal part 2c positioned midway between them. The central front abdominal part 2c corresponds to a central area of the present invention. A left breaking part 17a extending vertically through the front abdominal section 2 is formed between the left front abdominal part 2a and central front abdominal part 2c, and a right breaking part 17b extending vertically through the front abdominal section 2 is formed between the right front abdominal part 2b and central front abdominal part 2c. A bonding part 18 to the front crotch part 4a of the crotch section 4 is formed in the central front abdominal part 2c as shown in Fig. 7. Bonding at the bonding part 18 is created with an adhesive such as a hot melt adhesive. The breaking parts 17a and 17b are formed by perforations as shown in Fig. 2. The front abdominal section 2 can be separated at the breaking parts 17a and 17b as shown in Figs. 3 to 6 by breaking the breaking parts 17a and 17b. Here, the breaking parts 17a and 17b may be formed linearly as shown in Fig. 2 or may be formed in curved lines according to necessity.

Waist elastic members 19a and 19b are attached in a laterally stretched state to the upper edges 12a and 12b of the left front abdominal part 2a and right front abdominal part 2b. Leg elastic members 20a and 20b are attached in a laterally stretched state to the lower edges 14a, 14b of the left front abdominal part 2a and right front abdominal part 2b. Body elastic members 21a and 21 b are attached in a laterally stretched state to areas of the left front abdominal part 2a and right front abdominal part 2b between the upper edges 12a and 12b and lower edges 14a and 14b of the left front abdominal part 2a and right front abdominal part 2b. Contraction and stretch of these elastic members 19a, 19b, 20a, 20b, 21a and 21b allows the front abdominal section 2 (particularly, left front abdominal part 2a and right front abdominal part 2b) to fit snugly about the abdominal area of a wearer.

Such front abdominal section 2 is formed by sandwiching the elastic members 19a, 19b, 20a, 20b, 21a and 21b between an interior-layer sheet 57 on the skin-facing side and an exterior-layer sheet 58 on the exterior side, as shown in Fig. 7.

An almost sheet-like left adhesive piece 22a and a right adhesive piece 22b are bonded to the edges of the left front abdominal part 2a and right front abdominal part 2b on the exterior side and on the side of the central front abdominal part 2c with an adhesive such as a hot melt adhesive. These adhesive pieces 22a and 22b are used for securing the pants 1 when breaking the breaking parts 17a and 17b, and are provided to straddle the left and right breaking parts 17a and 17b on the exterior side of the front abdominal section 2. An adhesive part 23 corresponding to a first adhesive part to which the adhesive pieces 22a and 22b are to be attached is provided on the exterior side of the central front abdominal part 2c. This adhesive part 23 is formed continuously as a plane in a predetermined forming area A1. The lateral width Wl of this forming area A1 is set at or smaller than the distance D between the left and right breaking parts 17a and 17b (i.e., lateral dimension of central front abdominal part 2c) and the height W2 is set almost equal to the vertical dimension L1 of the adhesive pieces 22a and 22b.

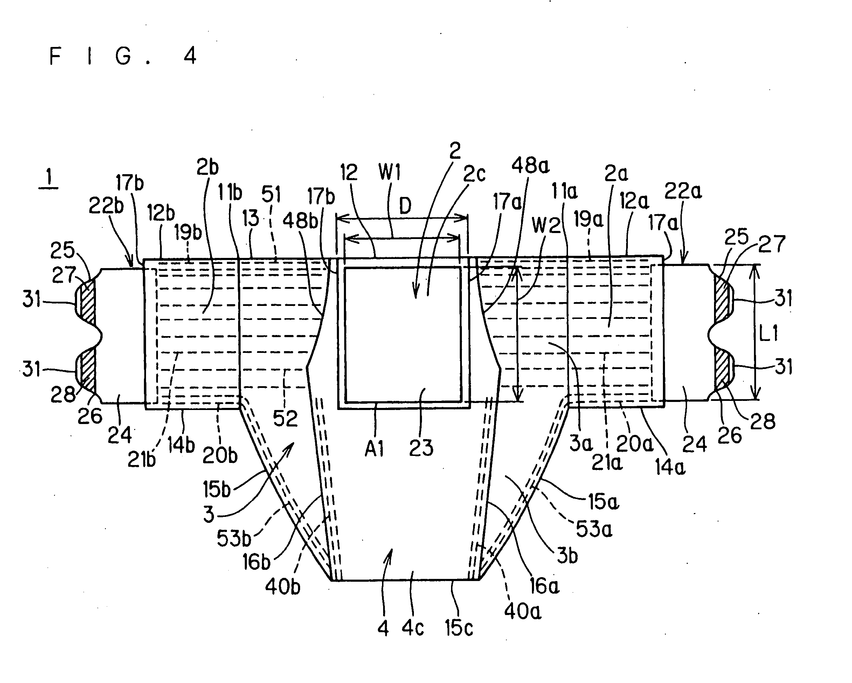

The adhesive pieces 22a and 22b, as shown in Fig. 4, each include an almost vertically long strip body 24 and two projections 25 and 26 bifurcated one on top of the other extending from the body 24 toward its free edge side. The body 24 has laterally outside edges bonded to the edges of the left front abdominal part 2a and right front abdominal part 2b on the side of the breaking parts 17a, 17b with an adhesive 66 such as a hot melt adhesive, as shown in Fig. 7.

The respective projections 25 and 26 of the adhesive pieces 22a and 22b are provided with adhesive parts 27 and 28, respectively, on the surface (skin-facing side) facing the central front abdominal part 2c. These adhesive parts 27 and 28 correspond to second adhesive parts. These adhesive parts 27 and 28 are attached to the adhesive part 23 provided on the central front abdominal part 2c freely detachably.

Specific examples of the adhesive part 23 may include a loop member having a nonwoven fabric, a woven fabric, a knitted material or the like with a fine loop structure being densely formed on its surface. Specific examples of the adhesive parts 27 and 28 may include a hook member with a fine hook structure in freely detachable engagement with the loop member being densely formed on its surface. More specifically, as the adhesive part 23, a plastic film composite material having on its surface a nonwoven fabric, a woven fabric or the like which is suitably used as a loop member for a hook-and-loop fastener is used for example. As the adhesive parts 27 and 28, a plastic film having pins densely formed on its surface which is suitably used as a hook member for a hook-and-loop fastener is used. Alternatively, a loop member may not be attached to the central front abdominal part 2c as a separate member, but the exterior surface of the central front abdominal part 2c may be surface-treated to serve as a loop member (adhesive part 23).

Alternatively, another specific example of the adhesive part 23 may include a plastic film or the like, which is surface-treated so as to have repetitive removability from an adhesive. Another specific example of the adhesive parts 27 and 28 may include a reusable adhesive by using PEELOIL, for example.

In the above-described two specific examples of the aforementioned adhesive part 23 and those of the adhesive parts 27 and 28, the structure on the adhesive part 23 side and the structure on the adhesive parts 27 and 28 side may be replaced with each other.

Further, according to the present embodiment, as shown in Figs. 7 and 8, the adhesive pieces 22a and 22b are provided with pleats 29 for folding the portions (specifically, the projections 25 and 26) on which the adhesive parts 27 and 28 are provided, to the skin-facing side. In such a folded state, the exterior side of portions (specifically, the projections 25 and 26) of the adhesive pieces 22a and 22b that are positioned on the free edge side with respect to the pleats 29 is temporarily fixed to the exterior side of the front abdominal section 2 (specifically, the central front abdominal part 2c) by temporarily fixing parts 30. That is, with the projections 25 and 26 folded to the skin-facing side, the exterior surface of the projections 25 and 26 facing outside when expanded and the exterior surface of the central front abdominal part 2c are temporarily fixed by the temporarily fixing parts 30.

The disposable pants 1 are shipped from a factory with the projections 25 and 26 folded and temporarily fixed in this manner.

As the temporarily fixing parts 30, means such as adhesion by heating welding, ultrasonic welding or an adhesive (e.g., hot melt adhesive) is used, and bonds the adhesive pieces 22a, 22b and the central front abdominal part 2c with such a strength that they can be peeled. Further, a temporarily fixing part 30 may be formed as a plurality of spotted bonding parts extending in the vertical direction, or may be formed as a linear bonding part extending in the vertical direction. While the temporarily fixing parts 30 are provided for each of the projections 25 and 26 in two lines extending in the vertical direction in the present embodiment, a temporarily fixing part 30 may be provided in a single line.

Further, the tips of the respective projections 25 and 26 of the adhesive pieces 22a and 22b are tabs 31 (cf. Fig. 4) for easy lifting of the adhesive pieces 22a and 22b. These tabs 31 are not provided with adhesive parts 27 and 28.

With such structure, the temporarily fixing parts 30 of the right adhesive piece 22b are peeled while holding the pleat 29 of the right adhesive piece 22b and pulling it outwardly to the right, the right breaking part 17b is broken as shown in Fig. 3, so that the right front abdominal part 2b is expanded to the right integrally with the right adhesive piece 22b. In the state shown in Fig. 3, the projections 25 and 26 of the right adhesive piece 22b are in the folded state, and turn into the state shown in Fig. 4 when expanded.

Similarly, the temporarily fixing parts 30 of the left adhesive piece 22a are peeled while holding the pleat 29 of the left adhesive piece 22a and pulling it outwardly to the left, the left breaking part 17a is broken as shown in Fig. 4, so that the left front abdominal part 2a is expanded to the left integrally with the left adhesive piece 22a. At this time, the central front abdominal part 2c is kept bonded to the crotch section 4 and remains on the exterior side of the front crotch part 4a. In the state shown in Fig. 4, the projections 25 and 26 of the adhesive pieces 22a and 22b are fully expanded.

Accordingly, in the disposable pants 1, as shown in Fig. 7, the front abdominal section 2 and crotch section 4 are secured by the bonding part 18 in the state at the time of shipping when the breaking parts 17a and 17b are yet to be broken. Therefore, they function as pants, and are easily raised/lowered similarly to typical disposable pants having no opening/closing means such as the adhesive pieces 22a and 22b, and the like.

Further, in the case where the disposable pants 1 are worn as pants and when an absorber 43 to be described later absorbs and contains bodily wastes, the temporarily fixing parts 30 of the adhesive pieces 22a and 22b may be peeled to break the breaking parts 17a and 17b, so that the pants 1 can easily be removed from the wearer. In this case, the pants 1 can be removed from the wearer without taking his/her garments off.

Further, after peeling the temporarily fixing parts 30 of the adhesive pieces 22a and 22b to break the breaking parts 17a, 17b and expand the front abdominal section 2 to see how the inside of the pants 1 gets soiled, the adhesive pieces 22a and 22b may be engaged with the adhesive part 23, to thereby return the pants 1 to its original state as pants. Further, when the pants 1 and an optional pad such as a urine pad are used in combination, the adhesive pieces 22a and 22b can be attached/detached to facilitate replacing such optional pad, and the like.

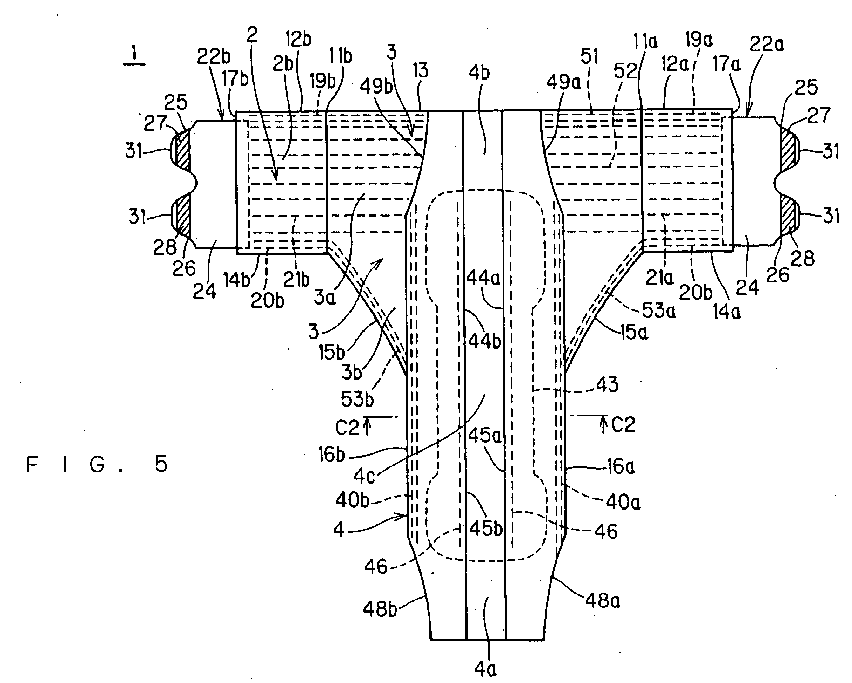

Further, the pants 1 may be used as a typical disposable diaper applying the pants, as expanded as shown in Fig. 5 before putting them on, around the wearer's hips and then closing them as shown in Figs. 4, 3 and 10 in this order, so that the pants 1 can be used as a typical disposable diaper. In this case, the pants 1 can be put on and removed without taking off wearer's garments.

### <Crotch Section>

The crotch section 4, as shown in Fig. 5, has an almost strip shape extending in the front-to-rear direction when expanded as an overall configuration. This crotch section 4 includes the front crotch part 4a, rear crotch part 4b and central crotch part 4c positioned midway between them, and is applied to the crotch of a wearer mainly setting the central crotch part 4c at the center. The front crotch part 4a, as shown in Figs. 5 to 7 and 9, is bonded to the central front abdominal part 2c by the bonding part 18 while overlapping the interior side of the central front abdominal part 2c. The rear crotch part 4b, as shown in Fig. 5, is bonded and fixed to the rear section 3 while overlapping the interior side of the rear section 3. Leg elastic members 40a and 40b are attached to the left edge 16a and right edge 16b of such crotch section 4 in a stretched state in the direction that the edges 16a and 16b extend.

The crotch section 4 is formed by sandwiching the absorber 43 between a liquid-permeable top sheet 41 and a liquid-impermeable backsheet 42. The absorber 43 has a predetermined width and extends in the front-to-rear direction in the form of strip with the central crotch part 4c set at the center. The both left and right sides of the absorber 43 on the interior side of the crotch section 4 are provided with standing parts 44a and 44b extending in the direction that the crotch section 4 extends.

For instance, the top sheet 41 is made of a liquid-permeable nonwoven fabric or the like, and the backsheet 42 is made of a water-repellant nonwoven fabric or the like. The absorber 43 is formed, for example, by covering a mass of a hydrophilic fiber assembly layer such as crushed pulp fibers or cellulose fibers mixed with a particulate gelling agent, with a covering sheet such as a sheet of paper like tissue paper, a liquid-permeable nonwoven sheet or the like, and is formed in a predetermined shape.

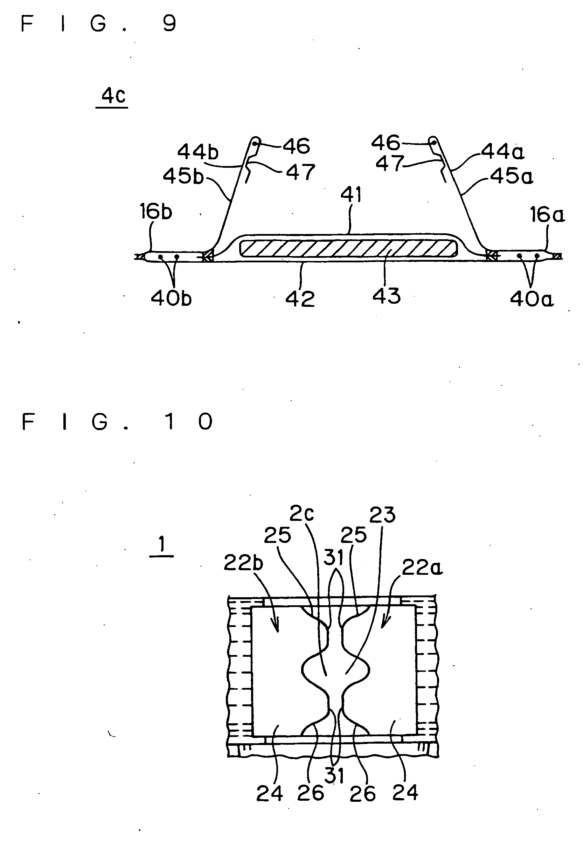

As to areas of the top sheet 41 and backsheet 42 that do not overlap the absorber 43, surfaces facing each other are bonded to each other with an adhesive such as a hot melt adhesive. More preferably, as shown in Fig. 9, the width of the top sheet 41 is determined to cover the skin-facing side of the absorber 43 and to be slightly narrower than the width of the backsheet 42, and portions of the top sheet 41 extending off the absorber 43 are bonded to the backsheet 42 with an adhesive such as a hot melt adhesive. Left and right side sheets 45a and 45b constituting the standing parts 44a and 44b are bonded to the skin-facing side of portions of the backsheet 42 extending off the top sheet 41 with an adhesive such as a hot melt adhesive.

Further, the both edges of the side sheets 45a and 45b in the front-to-rear direction are bonded to the both edges of the crotch section 4 in the front-to-rear direction with an adhesive 60 such as a hot melt adhesive. The laterally inside edges of the side sheets 45a and 45b are fixed by heating welding (or ultrasonic welding) or the like with sealing parts 47 so as to enclose elastic members 46 extending in the front-to-rear direction. The standing parts 44a and 44b have their laterally inside edges contracted by the contractive force of the elastic members 46, and are thereby raised in a direction to be pressed against the wearer's skin, as shown in Fig. 9.

Furthermore, in the present embodiment, as shown in Fig. 5, left and right edges of areas of the crotch section 4 that overlap the front abdominal section 2 and a waist zone 3a of the rear section 3 are sloped edges 48a, 48b, 49a and 49b, and have a gradually tapered width toward the edges in the front-to-rear direction.

These sloped edges 48a and 48b prevent the front crotch part 4a from being hitched to curl up, become bent or affect the wearer's skin when raising/lowering the disposable pants 1, which allows smooth raising/lowering.

Further, the contractive force of the rear section 3 generally tends to decrease in an area where the rear section 3 and crotch section 4 overlap. However, forming a trim area by the aforementioned sloped edges 49a and 49b gradually increases the area of the elastic part of the rear section 3 toward the top side of the rear section 3, which allows the rear section 3 to easily fit the wearer's back.

As an alternative structure of the crotch section 4, the absorber 43 may be adhered to the skin-facing side of the sheet 42, rather than sandwiching the absorber 43 between the sheets 41 and 42, and the sheet 41 may be omitted. Alternatively, the absorber 43 with sheets bonded to its front and rear edges may be used as the crotch section 4, or a large absorber 43 may be used as the crotch section 4 and the sheets 41 and 42 may be omitted.

### <Rear Section>

The rear section 3, as shown in Figs. 3 to 6, has such a form that, when expanded, left and right lower side corners of almost rectangle are cut almost diagonally, and is applied to an area from the waist to hips on the wearer's back. For this purpose, this rear section 3 includes the waist zone 3a in the form of almost laterally long strip in plan view mainly positioned on the waist on the wearer's back and a hip zone 3b of almost trapezoidal form in plan view joined downwardly to the waist zone 3a and mainly positioned on the wearer's hips. A waist elastic member 51 is attached in a laterally stretched state to the upper edge 13 of the waist zone 3a. A body elastic member 52 is attached in a laterally stretched state to the other area of the waist zone 3a. A leg elastic member 53a is attached to the sloped edge 15a on the left lower side of the rear section 3 in a stretched state along the edge 15a. A leg elastic member 53b is attached to the sloped edge 15b on the right lower side of the rear section 3 in a stretched state along the edge 15b. Contraction and stretch of these elastic members 51, 52, 53a and 53b allows the rear section 3 to easily fit the wearer's back and hips.

Particularly, the hip zone 3b of the rear section 3 is formed to have a gradually tapered width downwardly and the leg elastic members 53a and 53b provided on the left and right sloped edges 15a and 15b. Therefore, the hip zone 3b easily fits the wearer's hips when the edges 15a and 15b are contracted by the contractive forces of the leg elastic members 53a and 53b.

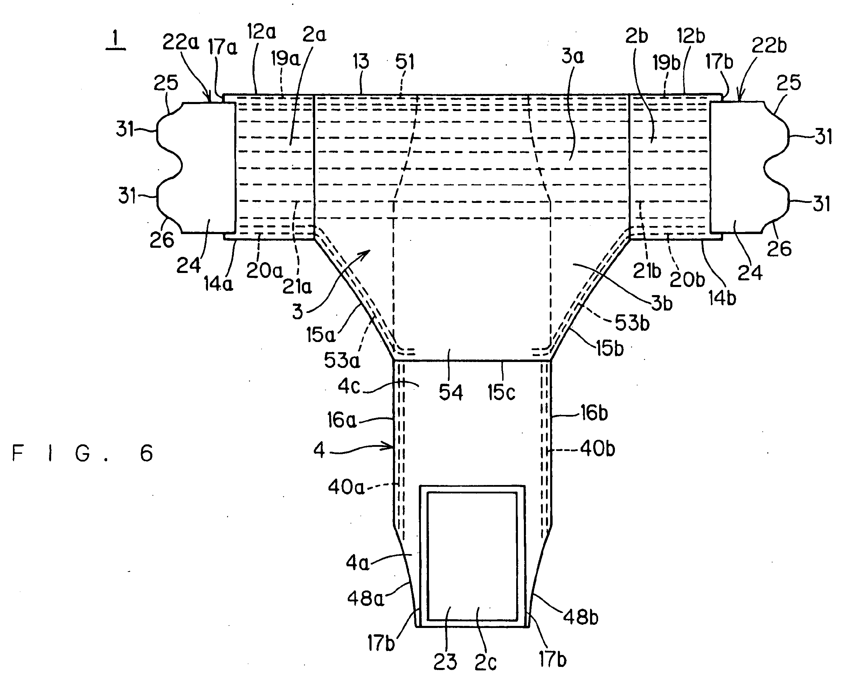

The leg elastic members 53a and 53b are continuously attached to the hip zone 3b along the left and right sloped edges 15a and 15b and lower edge 15c of the hip zone 3b, and then, at least an area 54 overlapping the absorber 43 of the crotch section 4 (cf. Fig. 6) is subjected to a weakening process. The weakening process is a process of cutting the elastic member in that area 54 or weakening its contractive force, or the like, to thereby bring about a no-tension state. This prevents the absorber 43 from causing an undesired contortion due to the contractive forces of the leg elastic members 53a and 53b and from degrading in its absorptive function.

### <Other Structure and Material for Respective Parts, etc.>

As to the left and right elastic members 19a, 19b, elastic members 20a, 20b, and elastic members 21a, 21 b provided in the front abdominal section 2, similarly to the case of the aforementioned leg elastic members 53a and 53b, it is preferable that the elastic members 20a, 20b, 21 a and 21 b be provided laterally continuously in the front abdominal section 2 through the central front abdominal part 2c, and then portions of the elastic members 20a, 20b, 21a and 21b positioned in the central front abdominal part 2c be subjected to the weakening process.

Further, the material for the adhesive pieces 22a and 22b may be selected appropriately from nonwoven fabrics, woven fabrics, knitted fabrics and plastic materials. Among them, a nonwoven fabric manufactured by one or a combination of a plurality of processes among spun-bond process, air-through process, point-bond process, melt-blow process and air-laid process is preferable. Further, a nonwoven fabric manufactured by a spun-bond process or SMS process combining the spun-bond process and melt-blow process with a weight of 30 to 100 g/m² is preferable in terms of strength. Most preferable is a nonwoven fabric manufactured by the spun-bond process with a weight of 50 to 85 g/m². The material can be selected appropriately from among synthetic fibers such as polypropylene, polyethylene, polyester, polyamide and the like and natural fibers such as pulp, silk and the like, but preferably, a synthetic fiber such as polypropylene, polyethylene or polyester can be used, and among them, one having a polypropylene or polyester fiber as its main component is strong and suitable. Most preferable one is a polyester fiber.

Further, for the elastic members 19a, 19b, 20a, 20b, 21a, 21b, 40a, 40b, 46, 53a and 53b, an elastic stretchable material (polyurethane thread, polyurethane film, natural rubber, etc.) typically used for disposable pants is employed, and is attached to a specified position of the pants 1 in a stretched state by adhering means such as a hot melt adhesive, heating welding, ultrasonic welding or the like.

As described above, the disposable pants 1 according to the present embodiment have functions of both pants and diaper, which are easy to raise/lower when putting them on.

Further, when the left and right adhesive pieces 22a and 22b are not used, holding the projections 25 and 26 of the adhesive pieces 22a and 22b in the folded state such as at the time of shipping from a factory can prevent the adhesive parts 27 and 28 of the adhesive pieces 22a and 22b from undesirably adhering to those places other than a specified position of the disposable pants 1 or adhering to garments and the like, which provides easy handling.

Further, when the adhesive pieces 22a and 22b are not used, the temporarily fixing parts 30 reliably allow the adhesive pieces 22a and 22b to be held in the state folded at the pleats 29 on the skin-facing side.

Further, when the adhesive pieces 22a and 22b are used, the temporarily fixing parts 30 can easily be peeled to expand the adhesive pieces 22a and 22b.

### <Variant>

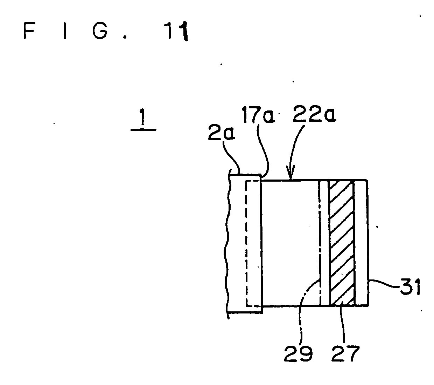

A Variant of the present embodiment may be as shown in Figs. 11.

In the variant shown in Fig. 11, the adhesive pieces 22a and 22b not provided with the projections 25 and 26 are disclosed. While Fig. 11 only shows the left adhesive piece 22a, the right adhesive piece 22b has a symmetric shape to the left adhesive piece 22a. In the case of the structure shown in Fig. 11, the adhesive part 27 is provided on the skin-facing side of the adhesive piece 22a, 22b on the free edge side with respect to the pleat 29, and the tab 31 is provided on the tip side portion with respect to the adhesive part 27. At the time of shipping from a factory, the adhesive pieces 22a and 22b are folded at the pleats 29 on the skin-facing side, and the temporarily fixing parts 30 hold the folded state, almost similarly to the structure shown in Fig. 8 or 10.

## Claims

1. Disposable pants (1) having:
a front abdominal section (2) and a rear section (3) joined annularly;
a crotch section (4) provided to be joined between the front abdominal section (2) and rear section (3), said crotch section (4) being provided with an absorber (43);
left and right breaking parts (17a, 17b) for breaking said front abdominal section (2) being provided on both left and right sides of an area of said front abdominal section (2) to which said crotch section (4) is joined;
a first adhesive part (23) provided in at least part of a central area positioned between said left and right breaking parts (17a, 17b) on an exterior side of said front abdominal section (2);
left and right adhesive pieces (22a, 22b) bonded to laterally outward sides of said left and right breaking parts (17a, 17b) in said front abdominal section (2); and
second adhesive parts (27, 28) provided on a skin-facing side of said left and right adhesive pieces (22a, 22b) to be attached/detached to/from said first adhesive part (23), and having functions of both pants and a diaper at once,
**characterized in that**
when a function of pants of said disposal pants (1) is used, a portion (25, 26) of each of the left and right adhesive pieces (22a, 22b) on which said second adhesive parts (27, 28) are provided is folded on the skin-facing side so as to prevent the adhesion of said second adhesive parts (27, 28), wherein
in such a folded state, temporarily fixing parts (30) provided on the respective skin-facing side of said portions (25, 26) holds the corresponding adhesive pieces (22a, 22b) in said folded state.

2. The disposable pants (1) according to claim 1, wherein
said temporarily fixing parts (30) bond said portions (25, 26) of said left and right adhesive pieces (22a, 22b) folded on the skin-facing side with such a strength that they can be peeled.

3. The disposable pants (1) according to claim 1 or 2, wherein
an elastic stretchable means is provided in an area other than said central area of said front abdominal section (2) and an area around the hips of said rear section (3).

## Patentansprüche

1. Einwegslips bzw. -höschen (1) mit:
einem vorderen Unterleibsabschnitt (2) und einem hinteren Abschnitt (3), die ringförmig verbunden sind;
einem Schrittabschnitt (4), der bereitgestellt ist, um zwischen dem vorderen Unterleibsabschnitt (2) und dem hinteren Abschnitt (3) angefügt zu werden, wobei der Schrittabschnitt (4) mit einem Absorber (43) versehen ist;
linken und rechten Abrissteilen (17a, 17b) zum Abreißen des vorderen Unterleibsabschnitts (2), die sowohl auf der linken als auch rechten Seite eines Bereichs des vorderen Unterleibsabschnitts (2) bereitgestellt sind, mit dem der Schrittabschnitt (4) verbunden ist;
einem ersten Klebstoffteil (23), der wenigstens in einem Teil eines Mittelbereichs bereitgestellt ist, der zwischen den linken und rechten Abrissteilen (17a, 17b) auf einer Außenseite des vorderen Unterleibsabschnitts (2) positioniert ist;
linken und rechten Klebstoffstücken (22a, 22b), die an seitliche Außenseiten der linken und rechten Abrissteile (17a, 17b) in dem vorderen Unterleibsabschnitt (2) geklebt sind; und
zweiten Klebstoffteilen (27, 28), die auf einer hautzugewandten Seite der linken und rechten Klebstoffstücke (22a, 22b) bereitgestellt sind, die an dem ersten Klebstoffteil (23) befestigt/von diesem gelöst werden sollen, und gleichzeitig sowohl Funktionen von Höschen als auch einer Windel haben,
**dadurch gekennzeichnet, dass**
wenn eine Funktion von Höschen der Einweghöschen (1) verwendet wird, jeweils ein Abschnitt (25, 26) der linken und rechten Klebstoffstücke (22a, 22b), auf dem die Klebstoffteile (27, 28) bereitgestellt sind, auf die hautzugewandte Seite gefaltet wird, um das Kleben der zweiten Klebstoffteile (27, 28) zu verhindern, wobei
in einem derartigen gefalteten Zustand provisorische Befestigungsteile (30), die auf der jeweiligen hautzugewandten Seite der Abschnitte (25, 26) bereitgestellt sind, die entsprechenden Klebstoffstücke (22a, 22b) in dem gefalteten Zustand halten.

2. Einweghöschen (1) nach Anspruch 1, wobei
die provisorischen Befestigungsteile (30) die Abschnitte (25, 26) der linken und rechten Klebstoffstücke (22a, 22b) mit einer derartigen Festigkeit auf der hautzugewandten Seite gefaltet halten, dass sie abgezogen werden können.

3. Einweghöschen (1) nach Anspruch 1 oder 2, wobei
ein elastisches dehnbares Mittel in einem Bereich außer dem mittleren Bereich des vorderen Unterleibsabschnitts (2) und einem Bereich des hinteren Abschnitts (3) um die Hüften herum bereitgestellt ist.

## Revendications

1. Des culottes jetables (1) ayant :
une section abdominale avant (2) et une section arrière (3) jointes de manière annulaire ;
une section d'entrejambe (4) destinée à réunir la section abdominale avant (2) et la section arrière (3), ladite section d'entrejambe (4) étant dotée d'un absorbeur (43) ;
des parties de rupture gauche et droite (17a, 17b) pour rompre ladite section abdominale avant (2) présentes sur les côtés gauche et droit d'une zone de ladite section abdominale avant (2) à laquelle ladite section d'entrejambe (4) est jointe ;
une première partie adhésive (23) présente dans au moins une partie de la zone centrale positionnée entre lesdites parties de rupture gauche et droite (17a, 17b) sur un côté extérieur de ladite section abdominale avant (2) ;
des pièces adhésives gauches et droites (22a, 22b) collées aux côtés latéralement extérieurs desdites parties de rupture gauches et droites (17a, 17b) dans ladite section abdominale avant (2) ; et
des deuxièmes parties adhésives (27, 28) présentes sur un côté face à la peau desdites pièces adhésives gauches et droites (22a, 22b) à attacher à/détacher de ladite première partie adhésive (23), et ayant des fonctions à la fois de culottes et de couche,
**caractérisées en ce que**
lorsqu'une fonction de culottes desdites culottes jetables (1) est utilisée, une portion (25, 26) de chacune des pièces adhésives gauches et droites (22a, 22b) sur laquelle lesdites deuxièmes parties adhésives (27, 28) sont présentes est repliée sur le côté face à la peau de manière à empêcher l'adhérence desdites deuxièmes parties adhésives (27, 28), dans laquelle
dans un tel état replié, des parties de fixation temporaires (30) présentes sur le côté face à la peau respectif desdites portions (25, 26) maintiennent les pièces adhésives correspondantes (22a, 22b) dans ledit état replié.

2. Les culottes jetables (1) selon la revendication 1, dans lesquelles lesdites parties de fixation temporaires (30) collent lesdites portions (25, 26) desdites pièces adhésives gauches et droites (22a, 22b) repliées sur le côté face à la peau avec une force telle qu'elles peuvent être pelées.

3. Les culottes jetables (1) selon la revendication 1 ou 2, dans lesquelles un moyen élastique extensible est fourni dans une zone autre que ladite zone centrale de ladite section abdominale avant (2) et une zone autour des hanches de ladite section arrière (3).
